# EUROPÄISCHE PATENTSCHRIFT

(11) **EP 1 338 237 B1**
(45) Veröffentlichungstag und Bekanntmachung des Hinweises auf die Patenterteilung: **06.09.2006**
(21) Anmeldenummer: 03002462.4
(22) Anmeldetag: 05.02.2003
(51) Int. Cl.: A61B 1/12, G01F 1/00

(54) **Verfahren und Vorrichtung zum Überprüfen der Durchgängigkeit von Endoskopkanälen**
Method and apparatus for testing the patency of endoscope channels
Méthode et appareil pour tester l'ouverture des canaux d'un endoscope

(30) Priorität: 26.02.2002 DE 10208035
(43) Veröffentlichungstag der Anmeldung: 27.08.2003
(73) Patentinhaber: BHT Hygienetechnik GmbH, 86316 Friedberg/Derching (DE)
(72) Erfinder: Annecke, Karl Heinz Dr., 64625 Bensheim (DE)
(74) Vertreter: von Bülow, Tam

(56) Entgegenhaltungen:
- EP-A- 0 072 257
- WO-A-91/18266
- GB-A- 2 275 341
- US-A- 3 271 993
- US-A- 4 395 918
- US-A- 4 906 165
- US-A- 5 738 824

## Beschreibung

Die Erfindung bezieht sich auf eine Vorrichtung zum Überprüfen der Durchgängigkeit von Endoskopkanälen gemäß dem Oberbegriff des Patentanspruches 1.

Eine solche Vorrichtung ist aus der US 5,738,824 A bekannt. Dort wird Flüssigkeit aus einem Flüssigkeitsbehälter mittels einer Pumpe unter Druck durch ein Durchflußprüfgerät und von dort durch einen oder mehrere zu überprüfende Endoskopkanäle geleitet. Die aus den Endoskopkanälen austretende Flüssigkeit wird wiederum durch ein Durchflußprüfgerät und von dort zurück in den Flüssigkeitsbehälter geleitet. An die Verbindungsleitung zwischen der Pumpe und dem Durchflußprüfgerät ist noch eine Druckstoßeinrichtung angeschlossen, die einen pneumatischen oder hydraulischen Druck erzeugen kann. Die Durchflußprüfgeräte messen das Volumen des in einer Zeiteinheit den Kanal durchströmenden Fluids. Dies kann nach einer Ausführungsform dadurch erfolgen, daß der Druckverlauf mit der Zeit gemessen und der ermittelte Druckabfall mit der Menge des den Kanal durchströmenden Fluids in Bezug gesetzt wird. Bei Fluiden mit vernachlässigbarer Kompressibilität ist der Druckabfall je Zeiteinheit direkt dem aus dem Flüssigkeitsbehälter austretenden Flüssigkeitsvolumen proportional.

Die GB 2 275 341 A beschreibt ein Verfahren zur Inspektion von hohlen Turbinenschaufeln, bei dem Wasser durch Kanäle der Turbinenschaufeln geleitet wird und die Durchflußrate gemessen wird. Dies erfolgt nach einem Ausführungsbeispiel mit einem Flüssigkeitsbehälter und Pegelsensoren, die den jeweiligen Flüssigkeitspegel in dem Behälter messen. Die Pegeländerung pro Zeiteinheit ist dann der Durchflußrate proportional.

Die EP 0 072 257 A2 beschreibt eine Vorrichtung zum Desinfizieren von Endoskopen. Sie weist mehrere mit Flüssigkeit befüllte Behälter auf, die mit Druck beaufschlagt sind, um die Flüssigkeit durch Endoskopkanäle zu drücken.

Die WO 91/18266 A zeigt eine Vorrichtung zum Messen des Volumens einer Flüssigkeit in einem Behälter, der mit Druckgas beaufschlagt ist. Im Behälter sind drei Drucksensoren angeordnet und zwar am Boden des Behälters, in vorbestimmter Höhe sowie an der Oberseite. Der Sensor am Boden mißt den hydrostatischen Druck zuzüglich dem Gasdruck. Der Sensor an der Oberseite des Behälters mißt nur den Gasdruck. Der dritte Sensor erkennt, wann der Flüssigkeitspegel auf einer vorbestimmten Höhe ist und dient zur Kalibrierung der beiden anderen Sensoren.

Die US 3,271,993 A betrifft die Eichung eines Durchflußreglers und verwendet einen Kalibriertank, der einen oberen und einen unteren Füllstandsgeber aufweist. Die Zeitdauer, innerhalb der sich der Flüssigkeitspegel von einem zum anderen Füllstandsgeber ändert, ist ein genaues Maß für die Durchflußrate.

Die US 4,395,918 A und die US 4,906,165 A zeigen Durchflußmesser mit einem Behälter bekannten Volumens. Mit zwei Füllstandsgebern können zwei vordefinierte Pegel und damit vordefinierte Volumina gemessen werden. Die Zeitdauer, innerhalb der sich der Pegel vom einen zum anderen Wert ändert, ist somit ein genaues Maß für eine Durchflußmenge.

Bei der maschinellen Reinigung, Desinfektion und Trocknung von Endoskopen muß sichergestellt werden, daß auch die sehr engen Endoskopkanäle vollständig gereinigt sind und nicht durch Fremdpartikel, die an der Kanalwand haften, ganz oder teilweise verstopft sind.

Aus der DE 44 23 730 C2 ist es bekannt, zum Reinigen von Endoskopen, die mindestens zwei Kanäle haben, einen Einsatzkörper mit steuerbaren Dichtungen zu verwenden, die so gesteuert werden, daß die einzelnen Kanäle individuell von Reinigungsflüssigkeit durchströmt werden. Hierdurch wird gewährleistet, daß jeder Kanal von der Reinigungsflüssigkeit durchströmt wird und nicht, wenn einer der Kanäle verstopft ist, die Reinigungsflüssigkeit über andere offene Kanäle abströmen kann.

Zur Verbesserung der Durchströmung von Endoskopkanälen schlägt die US 5,551,462 einen Druckerhöhungsapparat vor, der an die individuellen Endoskopkanäle angeschlossen ist und dafür sorgt, daß diese mit erhöhtem Druck einer Reinigungsflüssigkeit durchspült werden. Eine einwandfreie Überprüfung der vollständigen Durchgängigkeit der Endoskopkanäle ist damit jedoch nicht möglich.

Zur Überprüfung des Reinigungsergebnisses von Endoskopen schlägt die DE 299 03 174 U1 vor, zusätzlich zum Endoskop einen Prüfkörper in die Maschine einzulegen und mitzureinigen und anschließend den Prüfkörper zu untersuchen. Ist dieser einwandfrei gereinigt, so wird davon ausgegangen, daß auch das Endoskop einwandfrei gereinigt ist. Eine solche mittelbare Prüfung erlaubt jedoch keine sicheren Rückschlüsse auf die einwandfreie Reinigung und damit Durchgängigkeit von Endoskopkanälen.

Zur Messung der Durchgängigkeit von Kanälen wäre es möglich, ein unter Druck stehendes Medium (Flüssigkeit oder Gas) durch den Kanal zu leiten und mit einem Durchflußmesser die pro Zeiteinheit durch den Kanal fließende Menge des Mediums zu messen. Dies würde aber sehr genaue Durchflußmesser für kleinste Mengen erfordern und ist daher technisch sehr aufwendig.

Eine andere Möglichkeit der Durchflußmessung bestünde darin, eine vorbestimmte Menge eines Mediums pro Zeiteinheit durch den zu überprüfenden Kanal zu pressen und den dabei entstehenden Gegendruck zu messen. Bei Überschreiten eines vorgegebenen Druckwertes wird dann der Kanal als blockiert angesehen. In einer sehr einfachen Ausführungsform kann die Ermittlung der Blockierung dadurch erfaßt werden, daß eine den Druck erzeugende Pumpe aufgrund des Gegendruckes blockiert und einen deutlich höheren Strom zieht. Auch diese Ausführungsform ist jedoch technisch sehr aufwendig oder ungenau. Beide beschriebenen Verfahren erfordern darüber hinaus die individuelle Ankopplung jedes einzelnen Kanales an einen Durchflußmesser, Druckmesser bzw. eine Pumpe, die sich üblicherweise im Körper der Reinigungsmaschine befinden und nicht auf einem zur Beladung der Maschine mit dem Endoskop verwendeten Träger. Dies hat zur Folge, daß jedes Endoskop in der Waschkammer mit einer entsprechenden Anzahl von Anschlüssen, die heute üblicherweise zwischen fünf und zehn liegt, in der Maschine zu verbinden ist.

Allgemein sei noch darauf hingewiesen, daß mit der Erfindung nicht nur Kanäle von Endoskopen überprüft werden können, sondern die Durchgängigkeit aller Art von Kanälen in Geräten und Instrumenten, insbesondere also auch in sonstigen medizinischen Instrumenten.

Aufgabe der Erfindung ist es, Verfahren und Vorrichtung der eingangs genannten Art dahingehend zu verbessern, daß eine sichere Überprüfung der Durchgängigkeit von Endoskopkanälen gewährleistet ist und die individuelle Ankopplung der Kanäle des Endoskopes außerhalb der Waschkammer auf einem Träger erfolgt, wobei dieser Träger bei Einbringen in die Waschkammer so wenig wie möglich Ankopplungen benötigt.

Diese Aufgabe wird durch die in den Patentansprüchen 1 bzw. 2 angegebenen Merkmale gelöst. Vorteilhafte Ausgestaltungen und Weiterbildungen der Erfindung sind den Unteransprüchen zu entnehmen.

Das Grundprinzip der Erfindung liegt darin, daß eine vorbestimmte Flüssigkeitsmenge mit einem vorbestimmten Druck durch die zu überprüfenden Endoskopkanäle geleitet wird und die Zeitdauer hierfür gemessen und ausgewertet wird. Die vorgegebene Flüssigkeitsmenge stammt aus einem Behälter vorbekannter Größe, der sich auf dem Träger befindet. Sein Auslaß wird auf dem Träger mit dem jeweils zu überprüfenden Endoskopkanal verbunden. Die Flüssigkeitsoberfläche wird mit Druckluft beaufschlagt, die von der Maschine stammt. Somit ist beim Beschicken der Maschine lediglich der Behälter mit einer in der Maschine befindlichen Druckluftquelle zu koppeln. Pro Endoskopkanal ist auf dem Träger vorzugsweise ein individueller Behälter vorgesehen, wobei alle Behälter über eine gemeinsame Leitung mit der Druckluftquelle verbindbar sind, so daß auch nur für die Druckluftzufuhr eine Kupplung benötigt wird. Ebenso kann zum Befüllen der Behälter mit der Flüssigkeit eine gemeinsame Leitung vorgesehen sein, die somit ebenfalls nur eine Kupplung benötigt.

Schließlich benötigt man für jeden Behälter noch einen Sensor, der ermittelt, ob der Behälter leer ist. Hierfür können mechanische, optische, elektrische, pneumatische oder sonstige Sensoren verwendet werden, die in an sich bekannter Weise einen Flüssigkeitspegel messen oder einen Druck. Im letzteren Fall wird nämlich nach vollständiger Entleerung des Behälters nur noch Druckluft durch den Behälter und die angeschlossenen Endoskopkanäle fließen, was sich durch einen Druckabfall bemerkbar macht. Die Signale aller Sensoren können auf einer Leitung zusammengefaßt werden, so daß auch hierfür nur eine einzige Kupplung erforderlich ist.

Im folgenden wird die Erfindung anhand von Ausführungsbeispielen im Zusammenhang mit der Zeichnung ausführlicher erläutert. Es zeigt:
- Fig. 1: ein Prinzipschaltbild einer Vorrichtung zur Überprüfung der Durchgängigkeit eines Endoskopka- ' nales;
- Fig. 2: ein Prinzipschaltbild einer Vorrichtung nach der Erfindung, die für die Überprüfung mehrerer Endoskopkanäle ausgelegt ist;
- Fig. 3: eine Variante der Erfindung mit nur einem Behälter zur Überprüfung mehrerer Endoskopkanäle; und
- Fig. 4: ein Prinzipschaltbild der Vorrichtung mit einem Drucksensor zur Überwachung mehrerer Behälter.

Gleiche Bezugszeichen in den einzelnen Figuren bezeichnen gleiche bzw. funktionell einander entsprechende Teile.

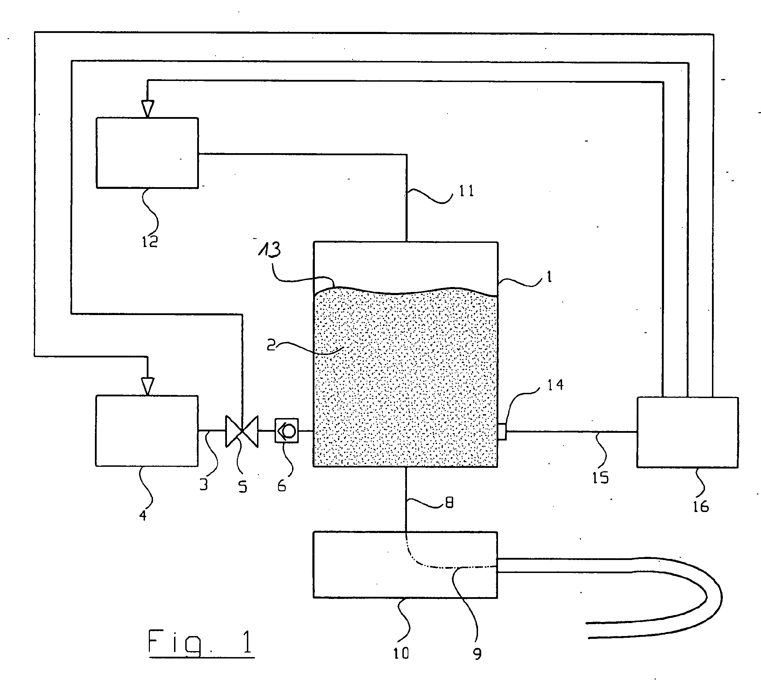

Zunächst sei auf Fig. 1 Bezug genommen. Dort ist ein Behälter 1 mit einem vorgegebenen Volumen gezeigt, der mit einer vorgegebenen Menge einer Flüssigkeit 2 befüllbar ist. Hierzu ist der Behälter 1 über eine Leitung 3 mit einer Flüssigkeitsversorgung 4 verbunden, wobei das Befüllen des Behälters 1 über ein steuerbares Ventil 5 erfolgen kann. In die Leitung 3 kann auch noch ein Rückschlagventil 6 eingesetzt sein, das verhindert, daß Flüssigkeit aus dem Behälter 1 zurück zur Flüssigkeitsversorgung fließen kann.

Im unteren Bereich des Behälters 1 ist ein Auslaß vorgesehen, der über eine Leitung 8 mit einem Kanal 9 eines Endoskopes 10 verbindbar ist.

Weiter ist der Behälter 1 über eine Leitung 11 mit einer Druckluftquelle 12 verbunden, mittels derer die Flüssigkeit 2 im Behälter 1 mit Druck beaufschlagbar ist. Die Leitung 11 mündet vorzugsweise in den oberen Bereich des Behälters 1, so daß die Oberfläche 13 der Flüssigkeit 2 mit Druckluft beaufschlagt wird. Im unteren Bereich des Behälters 1 ist ein Sensor 14 angebracht, der über eine Leitung 15 mit einer elektronischen Steuer- und Auswerteeinheit 16 verbunden ist. Diese im folgenden "Steuereinheit" genannte Einheit 16 überwacht das Ausgangssignal des Sensors 14 und steuert die Druckluftquelle 12 und die Flüssigkeitsversorgung 4 bzw. das Ventil 5.

Der Sensor 14 hat allgemein gesprochen die Aufgabe festzustellen, ob der Flüssigkeitspegel der Flüssigkeit 2 unter einem vorgegebenen Wert liegt und insbesondere, ob der Behälter 1 vollständig entleert ist. Hierzu können beispielsweise bekannte Füllstandsgeber verwendet werden, wie z.B. ein durch einen Schwimmer betätigter Schalter, ein elektronischer oder optischer Sensor, der das Vorhandensein einer Flüssigkeit feststellt. Es kann aber auch ein Drucksensor verwendet werden, wie weiter unten noch ausführlicher erläutert wird. '

Der Prozeßablauf der in Fig. 1 dargestellten Vorrichtung ist wie folgt:

In einem ersten Schritt wird der Behälter 1 vollständig mit Flüssigkeit gefüllt. Hierzu kann beispielsweise die Steuereinheit 16 eine nicht dargestellte Pumpe der Wasserversorgung 4 oder das Ventil 5 einschalten. Luft und überschießende Flüssigkeit aus dem Behälter können über die Leitung 11 und die Druckluftversorgung 12 entweichen.

In einem zweiten Schritt wird die Flüssigkeit 2 durch Druckluft aus der Druckluftquelle 12 durch den angeschlossenen Endoskopkanal 9 gepreßt. Ist alle Flüssigkeit 2 aus dem Behälter 1 durch den Kanal 9 gepreßt, so wird der Zustand "Behälter leer" von dem Sensor 14 erfaßt und an die Steuereinheit 16 gemeldet. Die Steuereinheit 16 mißt dabei die Zeitdauer, die benötigt würde, um sämtliche Flüssigkeit aus dem Behälter 1 durch den Endoskopkanal 9 zu pressen. Bei freiem Endoskopkanal 9, bekanntem Querschnitt des Endoskopkanals, bekanntem Volumen des Behälters 1 und bekanntem, vorzugsweise konstantem Druck der Druckluftquelle 12 muß der Behälter innerhalb einer vorgegebenen Zeitdauer vollständig entleert sein. Ist dies nicht der Fall, so ist dies ein sicheres Zeichen dafür, daß der Endoskopkanal 9 ganz oder teilweise blockiert ist.

In einem weiteren Schritt wird der Behälter 1 über die Leitung 3 wieder mit Flüssigkeit befüllt, wobei die Entlüftung über die Druckluftleitung 11 und die Druckluftversorgung 12 geschlossen ist, so daß die Flüssigkeit direkt in den Kanal des Endoskopes strömt und diesen reinigt bzw. desinfiziert.

Zum Ausblasen bzw. Trocknen der Kanäle wird die Flüssigkeitszufuhr abgeschaltet, beispielsweise durch Schließen des Ventiles 5 und dem Behälter 1 wiederum Druckluft aus der Druckluftquelle 12 zugeführt. Diese Druckluft kann ggf. auch vorgewärmt sein. Sie strömt dann für einen weiteren vorgegebenen Zeitraum durch den Kanal 9 des Endoskopes und bläst diesen aus und trocknet ihn. Alternativ kann die Trocknungsluft, die eventuell auch vorgewärmt sein kann, direkt über den Wasserkanal 3 zugeführt werden.

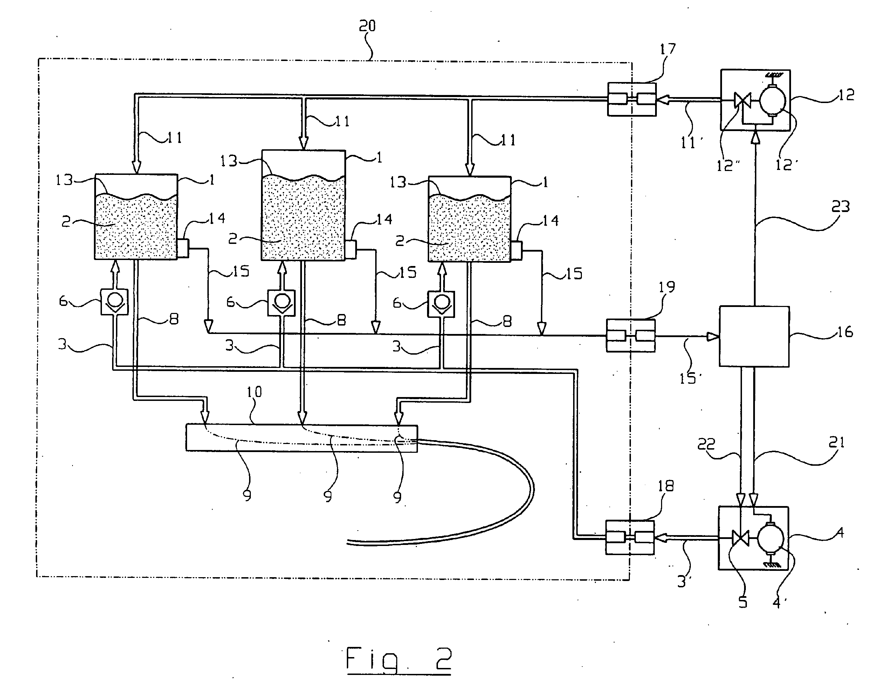

Fig. 2 zeigt ein Ausführungsbeispiel für ein Endoskop 10 mit drei Kanälen 9. Jedem Kanal 9 ist ein Behälter 1 zugeordnet und über die Leitung 8 an den jeweiligen Kanal 9 angeschlossen. Das Endoskop 9 und alle Behälter 1 sind hier auf einem gemeinsamen Träger 20 angeordnet, der beispielsweise ein Einsatzwagen sein kann, der in eine nicht dargestellte Reinigungs- und Desinfektionsmaschine eingeschoben werden kann. Der Träger hat hier drei Kupplungen 17, 18 und 19, wobei die Kupplung 17 die Druckluftleitungen 11 mit der Druckluftquelle 12 verbindet, die Kupplung 18 die Flüssigkeitszufuhrleitungen 3 mit der Flüssigkeitsversorgung 4 und schließlich die Kupplung 19 die Leitungen 15 und damit die Sensoren 14 mit der Steuereinheit 16 verbindet. Entsprechend dem in Fig. 1 dargestellten Ausführungsbeispiel sind alle Behälter 1 über je ein Rückschlagventil 6 mit der Wasserversorgung 4 sowie über die Leitungen 11 mit der Druckluftquelle 12 verbunden. Die Steuereinheit 16 ist darüber hinaus über elektrische Leitungen 21 und 22 mit der Flüssigkeitsversorgung verbunden, die hier beispielsweise eine Pumpe 4' und das Absperrventil 5 verfügt. Weiter ist die Steuereinheit 16 über eine elektrische Leitung 23 mit der Druckluftquelle 12 verbunden, die beispielsweise einen elektrisch angetriebenen Kompressor 12' und ggf. ebenfalls ein Absperrventil 12" aufweist.

Das Endoskop 10 kann somit noch außerhalb der Reinigungs- und Desinfektionsmaschine auf dem Träger 20 über die Leitungen 8 mit den einzelnen Behältern 1 verbunden werden, wobei hier darauf hinzuweisen ist, daß heutige Endoskope bis zu zehn oder mehr Kanäle aufweisen, die einzeln anzuschließen sind. Der Träger 20 muß dagegen nur über die drei Kupplungen 17, 18 und 19 mit Anschlüssen in der Maschine verbunden werden, was entweder von Hand oder auch über automatische Kupplungen erfolgen kann. Die der Maschine zugeordneten Leitungen sind mit 3', 11' und 15' bezeichnet.

Die Arbeitsweise der Vorrichtung nach Fig. 2 entspricht ansonsten vollständig der in Zusammenhang mit Fig. 1 beschriebenen. Weiter ist noch darauf hinzuweisen, daß die einzelnen Behälter 1 unterschiedliche Größe haben und damit an die Querschnitte der zu prüfenden Kanäle anzupassen sind. Damit können auch die Ausgangssignale der Sensoren 14 miteinander kombiniert werden, so daß ein Endoskop nur dann als einwandfrei akzeptiert wird, wenn in der vorgegebenen Zeit alle Behälter mit den auf dem jeweiligen Kanalquerschnitt angepaßten Volumina durch die Druckluft definierten Druckes geleert worden sind. Welcher der einzelnen Kanäle dabei noch blockiert ist, braucht dabei nicht erfaßt oder gemeldet zu werden, da bereits ein blockierter Kanal eine Fehlermeldung auslösen soll.

Zur weiteren Vereinfachung kann auch von bestimmten mittleren Querschnitten bestimmter Kanalgruppen ausgegangen werden, was zwar die absolute Genauigkeit der Aussage "blockiert" oder "frei" etwas verschlechtert, bei Einstellung der einzelnen Grenzwerte jedoch immer noch ein ausreichend genaues Ergebnis gibt. Damit können die Volumina der Behälter auch "gruppenweise" definiert werden und müssen nicht an jeden Kanal individuell angepaßt werden. Darüber hinaus besteht auch die Möglichkeit, die zulässige Entleerungszeit der Behälter individuell oder gruppenweise festzulegen und auch an unterschiedliche Endoskoptypen anzupassen.

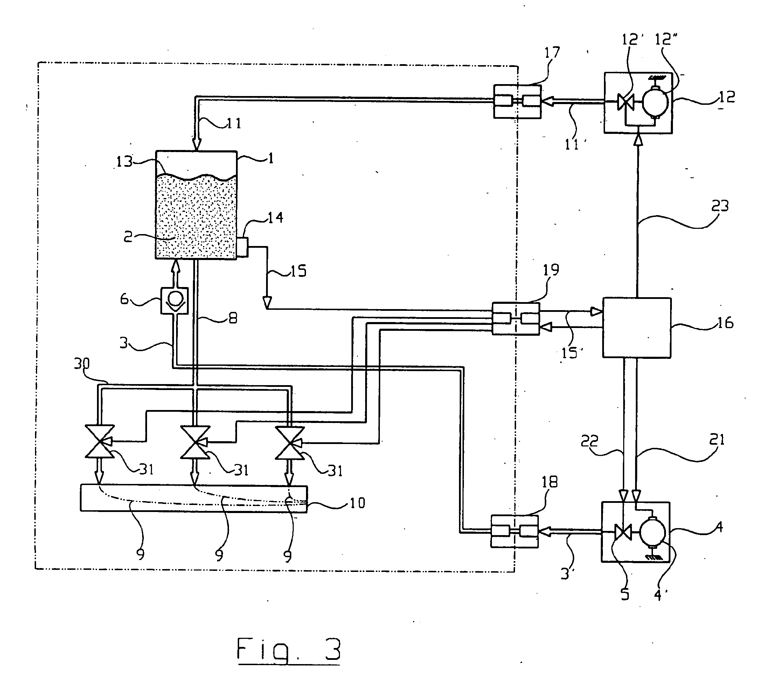

Weiter sei darauf hingewiesen, daß es auch möglich ist, mehrere Kanäle mit nur einem Behälter 1 zu überprüfen. Hierzu wird - wie in Fig. 3 gezeigt - die Leitung 8 des Behälters 1 mit einer Leitungsverzweigung 30 verbunden, die eine Vielzahl von Ausgangsleitungen aufweist, die individuell durch ein steuerbares Absperrventil 31 mit den einzelnen Endoskopkanälen verbunden sind. Die Steuereinheit 16 öffnet dann zeitlich nacheinander jeweils eines der Ventile 31 und führt so zeitlich nacheinander die Überprüfung der einzelnen Kanäle durch. Jeweils vor der Überprüfung eines Kanales wird - wie oben beschrieben -der Behälter 1 neu gefüllt.

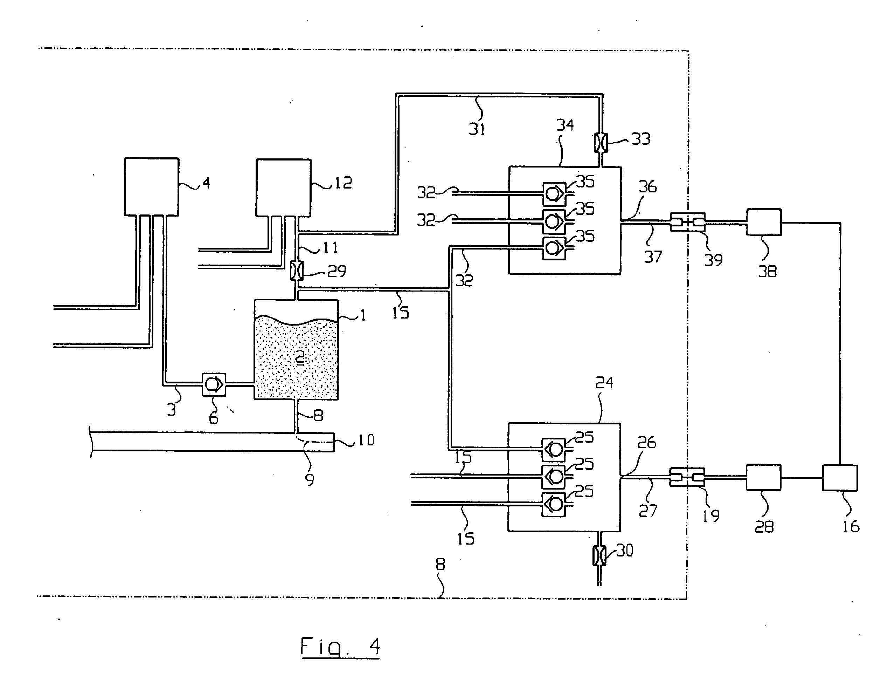

Fig. 4 zeigt eine Variante der Erfindung, bei der das Signal "Behälter leer" durch einen Drucksensor 28 ermittelt wird. In der Druckluftleitung 11 zum Behälter 1 ist eine Strömungsverengung 29 angeordnet, die die Menge der zugeführten Druckluft begrenzt und somit einen sicheren stabilen Druck im Behälter gewährleistet. In dem Moment, zu dem die gesamte Flüssigkeit durch den Kanal des Endoskopes entwichen ist, sinkt der Druck im Behälter ab, da der Kanal des Endoskopes der jetzt nachströmenden Luft einen sehr viel geringeren Widerstand entgegensetzt als zuvor der Flüssigkeit. Dieser Druckabfall wird durch die Leitung 15 an ein Element 24 weitergegeben, das die Sensoranschlüsse aller Behälter logisch "ODER" verknüpft, so daß der Druck am Ausgang 26 des Elementes 24 nur dann absinkt, wenn alle Eingangsdrücke abgesunken sind. Dieser Ausgang wird über die Kupplung 19 des Trägers 20 zu dem Drucksensor 28 weitergeleitet, der ein elektrisches Signal an die Steuereinheit 16 abgibt. Das Element 24 besteht im vorliegenden gezeigten Ausführungsbeispiel aus mehreren Rückschlagventilen 25, die jeweils über eine Leitung 15 bzw. einen Schlauch an einen zugeordneten Behälter 1 sowie an eine Entlüftung mit definiert kleinem Querschnitt angeschlossen sind. Die Ausgänge der Rückschlagventile sind miteinander zu dem Ausgang 26 verbunden. Der höchste der anstehenden Drücke auf den Leitungen 15 wird damit zu dem Drucksensor 28 weitergeleitet.

Durch die Rückschlagventile ist aber sichergestellt, daß der höhere Druck eines der Behälter nicht zu einem anderen Behälter gelangt, der schon niedrigeren Druck hat. Erst wenn alle Behälter den niedrigeren Druck haben, spricht der Drucksensor 28 auf die Druckabsenkung an und meldet dies an die Steuereinheit 16.

Die Einheit 24 ist im Bereich des Deckels des Behälters 1 bzw. in der Leitung 11 nach der Strömungsverengung 29 angeschlossen, damit möglichst wenig Feuchtigkeit in Richtung zu der Einheit 24 wandert und deren Funktion behindert. Die Ausgänge der Rückschlagventile 25 enden offen in der Einheit 24, die hier ein Behälter ist und dessen Auslaß 26 einen definierten Querschnitt hat.

Natürlich können an den Behältern 1 auch jeweils individuelle Drucksensoren entsprechend den Sensoren 14 der Fig. 1 angebracht sein, deren Signale dann - wie vorher beschriebenindividuell oder zusammengefaßt zur Steuereinheit 16 geführt werden. Weiter kann mit Drucksensoren festgestellt werden, ob ein Anschluß 8 an einem Endoskop 10 angeschlossen ist oder offenliegt, d.h. eventuell nicht fachgerecht verbunden wurde. Hierzu werden alle Anschlüsse 8 der Vorrichtung mit einem Auslaß definierten Querschnitts verbunden, der einen durchschnittlichen Endoskopkanal simuliert. Nicht sachgerecht angeschlossene Anschlüsse 8 führen dann dazu, daß sich der zugeordnete Behälter in sehr viel kürzerer Zeit entleert, was durch den Sensor 14 (Fig. 1) registriert wird. Auf diese Weise werden offene Verbindungen an den Anschlüssen 8 erkannt und zwar entweder individuell oder wiederum durch eine Kombination aller Signale als pauschale Fehlermeldung, die den Anwender informiert.

Diese Funktion wird durch ein Element 34 realisiert, das ähnlich wie das Element 24 aufgebaut ist, allerdings eine logische "UND"-Funktion hat. Die Eingänge 32 dieses Elementes 34 sind mit der Leitung 15 verbunden, an der der Eingangsdruck zum Behälter 1 anliegt und zwar gemessen hinter der Strömungsverengung 29. Weiter ist an den Innenraum des Elementes 34 eine Leitung 31 angeschlossen, die mit dem Druckerzeuger 12 bzw. der Leitung 11 verbunden ist und zwar vor der Strömungsverengung 29. Auch in die Leitung 31 ist eine Strömungsverengung 33 zwischengeschaltet. Der Ausgangsdruck am Ausgang 36 der Einheit 34 entspricht damit immer dem niedrigsten Druck aller Eingangsdrücke und wird über eine weitere Kupplung 39 zu einem weiteren Drucksensor 38 geleitet und von dort zur Steuereinheit 16. Die Einheit 34 enthält ebenfalls Rückschlagventile 35, die im Gegensatz zur Einheit 24 allerdings in umgekehrter Richtung geschaltet sind. Über die Leitung 31 und die Strömungsverengung 33 gelangt Druckluft unmittelbar vom Drucklufterzeuger 12 in den Innenraum der Einheit 34. Solange noch Flüssigkeit in den an die Eingänge 32 angeschlossenen Behälter ist, strömt nur sehr wenig Druckluft durch die Rückschlagventile 35, zumal die Druckluftzufuhr durch die Strömungsverengung 33 noch begrenzt ist. Ist an den Behälter 1 kein Endoskopkanal angeschlossen, so entweicht die dort befindliche Flüssigkeit recht schnell und anschließend auch die aus der Einheit 34 über die Leitungen 32 und 15 nachgespeiste Druckluft. Im Innenraum der Einheit 34 wird dann der Druck abfallen und der Drucksensor 39 spricht auf diesen Druckabfall an. Am Ausgang 36 der Einheit 34 liegt also nur dann ein hoher Druck an, wenn an allen Eingängen 32 noch ein hoher Gegendruck vorhanden ist. Insoweit findet also eine "UND"-Verknüpfung statt.

## Patentansprüche

1. Vorrichtung zum Überprüfen der Durchgängigkeit von Endoskopkanälen
- mit einem Flüssigkeitsbehälter (1), dessen Auslaß (8) mit dem zu überprüfenden Kanal (9) verbindbar ist,
- mit einer Druckluftquelle (12), die die Flüssigkeit (2) mit einem vorbestimmten Druck beaufschlagt,
- mit einem Drucksensor (28) und
- mit einer Steuereinheit (16),
**dadurch gekennzeichnet,**
**daß** der Flüssigkeitsbehälter (1) ein vorbestimmtes Volumen hat,
**daß** die Druckluftquelle (12) an den Flüssigkeitsbehälter (1) angeschlossen ist,
**daß** in der Druckluftzuleitung (11) zu dem Flüssigkeitsbehälter (1) eine Strömungsverengung (29) vorgesehen ist,
**daß** der Drucksensor (28) dazu geeignet ist zu erfassen, ob der Flüssigkeitsspiegel in dem Flüssigkeitsbehälter (1) unter einen vorbestimmten Wert gesunken ist, und dann ein Signal "Behälter leer" ausgibt, wenn der Druck im Flüssigkeitsbehälter (1) unter den vorbestimmten Wert gesunken ist, und
**daß** die Steuereinheit (16) dazu geeignet ist die Zeitdauer zu messen, innerhalb der das vorbestimmte Volumen des Flüssigkeitsbehälters (1) durch den zu überprüfenden Kanal (9) geflossen ist.

2. Vorrichtung nach Anspruch 1, **dadurch gekennzeichnet,**
**daß** der Flüssigkeitsbehälter (1) und das zu überprüfende Endoskop (10) auf einem gemeinsamen Träger (20) angeordnet sind und daß dieser Träger (20) Kupplungen (17, 18, 19) zur Ankupplung an eine Reinigungs- und Desinfektionsmaschine aufweist.

3. Vorrichtung nach Anspruch 2, **dadurch gekennzeichnet,**
**daß** mehrere Behälter (1) vorgesehen sind, die jeweils einem zu überprüfenden Kanal (9) oder eine Gruppe von Kanälen zugeordnet sind.

4. Vorrichtung nach Anspruch 3, **dadurch gekennzeichnet,**
**daß** die Behälter unterschiedliche Volumina aufweisen.

5. Vorrichtung nach Anspruch 3 oder 4, **dadurch gekennzeichnet,**
**daß** alle Behälter (1) an eine gemeinsame Druckluftquelle (12) anschließbar sind.

6. Vorrichtung nach einem der Ansprüche 1 bis 5, **dadurch gekennzeichnet,**
**daß** in einer Druckluftleitung (11), die den bzw. die Behälter (1) mit der Druckluftquelle (12) verbindet, ein steuerbares Ventil (12') zwischengeschaltet ist.

7. Vorrichtung nach Anspruch 3, **dadurch gekennzeichnet,**
**daß** ein gemeinsamer Drucksensor (28) für alle Behälter (1) vorgesehen ist, der nur dann ein Signal "Behälter leer" erzeugt, wenn der Druck in allen Behältern unter einen vorgegebenen Wert gesunken ist.

8. Vorrichtung nach Anspruch 7, **dadurch gekennzeichnet,**
**daß** alle Behälter (1) je über eine Leitung (15) und einen Leitungsverteiler (24) mit Rückschlagventilen (25) an den einen Sensor (28) angeschlossen sind.

9. Vorrichtung nach einem der Ansprüche 1 bis 8, **dadurch gekennzeichnet,**
**daß** eine gemeinsame Wasserversorgung (4) für das Befüllen der Behälter (1) vorgesehen ist.

10. Vorrichtung nach Anspruch 9, **dadurch gekennzeichnet,**
**daß** in Zuleitungen (3) von der Wasserversorgung (4) zu dem Behälter (1) je ein Rückschlagventil (6) angeordnet ist.

11. Vorrichtung nach einem der Ansprüche 1 bis 10, **dadurch gekennzeichnet,**
**daß** die Steuereinheit (16) die Wasserversorgung (4) und die Druckluftquelle (12) sowie ggf. zugehörige Ventile (5, 12') steuert und den Sensor (14, 28) bzw. die Sensoren (14) überwacht.

12. Vorrichtung nach einem der Ansprüche 1 bis 11, **dadurch gekennzeichnet,**
**daß** ein Sensor (38) vorgesehen ist, der überwacht, ob alle Behälter (1) an einen Kanal (9) angeschlossen sind.

13. Vorrichtung nach Anspruch 12, **dadurch gekennzeichnet,**
**daß** der Sensor (38) ein Drucksensor ist, der nur dann ein Signal "alle Behälter angeschlossen" erzeugt, wenn an allen Behältern ein vorgegebener Mindestdruck anliegt.

## Claims

1. Device for checking the patency of endoscope channels
- with a liquid container (1) of which the outlet (8) is capable of being connected to the channel (9) that is to be checked,
- with a compressed air source (12) which subjects the liquid (2) to a predetermined pressure,
- with a pressure sensor (28), and
- with a control unit (16),
**characterized in**
**that** the liquid container (1) has a predetermined volume,
**that** the compressed air source (12) is connected to the liquid container (1),
**that** a flow constriction (29) is provided in the compressed air line (11) to the liquid container (1), that the pressure sensor (28) is adapted to determine whether the surface of liquid in the liquid container (1) has dropped below a predetermined value, and then generates a signal "container empty" when the pressure in the liquid container (1) has dropped below a predetermined value, and
**that** the control unit (16) is able to measure the period of time within which the predetermined volume of the liquid container (1) is flowed through the channel (9) that is to be checked.

2. Device in accordance with Claim 1, **characterized in that** the liquid container (1) and the endoscope (10) that is to be checked are arranged on a common support (20), and that this support (20) has connectors (17, 18, 19) for connecting to a cleaning and disinfection machine.

3. Device in accordance with Claim 2, **characterized in**
**that** several containers (1) are provided that are each assigned to a channel (9) that is to be checked or a group of channels.

4. Device in accordance with Claim 3, **characterized in**
**that** the containers have different volumes.

5. Device in accordance with Claim 3 or 4, **characterized in**
**that** all containers (1) are capable of being connected to a common compressed air source (12).

6. Device in accordance with one of Claims 1 to 5, **characterized in**
**that** a controllable valve (12') is serially connected within a compressed air line (11) that connects the container (1) respectively the containers (1) to the compressed air source (12).

7. Device in accordance with Claim 3, **characterized in**
**that** a common pressure sensor (28) is provided for all the containers (1) that generates the signal "container empty" only when the pressure in all the containers has dropped below a predetermined value.

8. Device in accordance with Claim 7, **characterized in**
**that** the containers (1) are in each case all connected via non-return valves (25) to the one sensor (28) via a line (15) and a line distributor (24).

9. Device in accordance with one of Claims 1 to 8, **characterized in**
**that** a common water supply (4) is provided for filling the containers (1).

10. Device in accordance with Claim 9, **characterized in**
**that** a non-return valve (6) is in each case arranged in the supply lines (3) from the water supply (4) to the container (1).

11. Device in accordance with one of Claims 1 to 10, **characterized in**
**that** the control unit (16) controls the water supply (4) and the compressed air source (12) as well as the associated valves (5, 12') if required, and it monitors the sensor (14, 28) or the sensors (14).

12. Device in accordance with one of Claims 1 to 11, **characterized in**
**that** a sensor (38) is provided that monitors whether all the containers (1) have been connected to a channel (9) .

13. Device in accordance with Claim 12, **characterized in**
**that** the sensor (38) is a pressure sensor that generates the signal "all containers connected" only when a predefined minimum pressure prevails at all the containers.

## Revendications

1. Appareil pour tester l'ouverture des canaux d'un endoscope
- avec un réservoir à liquide (1) dont la sortie (8) peut être reliée au canal à tester (9),
- avec une source à air comprimé (12) qui amène le liquide (2) à une pression prédéterminée,
- avec un capteur de pression (28) et
- avec un boîtier de commande (16),
**caractérisé**
**en ce que** le réservoir à liquide (1) a un volume prédéterminé,
**en ce que** la source à air comprimé (12) est raccordée au réservoir à liquide (1),
**en ce qu'**un étranglement (29) est prévu dans la canalisation d'alimentation à air comprimé (11) conduisant au réservoir à liquide (1),
**en ce que** le capteur de pression (28) est approprié pour détecter si le niveau de liquide dans le réservoir à liquide (1) est descendu en-dessous d'une valeur prédéterminée et si un signal "réservoir vide" est alors émis lorsque la pression dans le réservoir à liquide (1) est descendue en-dessous de la valeur prédéterminée, et
**en ce que** le boîtier de commande (16) est approprié pour mesurer le temps que met le volume prédéterminé du réservoir à liquide (1) pour traverser le canal à tester (9).

2. Appareil selon la revendication 1, **caractérisé en ce que** le réservoir à liquide (1) et l'endoscope à tester (10) sont disposés sur un support commun (20) et **en ce que** ce support (20) présente des coupleurs (17, 18, 19) pour le couplage à un dispositif de nettoyage et de désinfection.

3. Appareil selon la revendication 2, **caractérisé en ce que** plusieurs réservoirs (1) sont prévus, qui sont respectivement affectés à un canal à tester (9) ou à un groupe de canaux.

4. Appareil selon la revendication 3, **caractérisé en ce que** les réservoirs présentent des volumes divers.

5. Appareil selon la revendication 3 ou 4, **caractérisé**
**en ce que** tous les réservoirs (1) peuvent être raccordés à une source à air comprimé commune (12).

6. Appareil selon l'une quelconque des revendications 1 à 5, **caractérisé**
**en ce qu'**une soupape gouvernable (12') est intercalée dans une canalisation à air comprimé (11), qui relie le et/ou les réservoir(s) (1) à la source à air comprimé (12).

7. Appareil selon la revendication 3, **caractérisé en ce qu'**un capteur de pression commun (28) est prévu pour tous les réservoirs (1), lequel n'émet un signal "réservoir vide" que lorsque la pression dans tous les réservoirs est descendue en-dessous d'une valeur prédéterminée.

8. Appareil selon la revendication 7, **caractérisé en ce que** tous les réservoirs (1) sont respectivement raccordés à l'un des capteurs (28) au moyen d'une canalisation (15) et d'un répartiteur de canalisations (24) avec des soupapes anti-retour (25).

9. Appareil selon l'une quelconque des revendications 1 à 8, **caractérisé**
**en ce qu'**une alimentation commune en eau (4) est prévue pour le remplissage des réservoirs (1).

10. Appareil selon la revendication 9, **caractérisé en ce qu'**une soupape anti-retour (6) est disposée respectivement dans des conduites d'alimentation (3) allant de l'approvisionnement en eau (4) jusqu'au réservoir (1).

11. Appareil selon l'une quelconque des revendications 1 à 10, **caractérisé**
**en ce que** le boîtier de commande (16) commande l'approvisionnement en eau (4) et la source à air comprimé (12) ainsi que, le cas échéant, des soupapes associées (5, 12') et contrôle le capteur (14, 28) et/ou les capteurs (14).

12. Appareil selon l'une quelconque des revendications 1 à 11, **caractérisé**
**en ce qu'**un capteur (38) est prévu, lequel contrôle si tous les réservoirs (1) sont raccordés à un canal (9).

13. Appareil selon la revendication 12, **caractérisé en ce que** le capteur (38) est un capteur de pression qui n'émet un signal "tous réservoirs raccordés" que lorsque la pression dans tous les réservoirs est une pression minimale prédéterminée.
